# EUROPEAN PATENT APPLICATION

(11) **EP 2 695 632 A1**
(43) Date of publication of application: **12.02.2014**
(21) Application number: 12005814.4
(22) Date of filing: 10.08.2012
(51) Int. Cl.: A61M 25/00

(54) **Fluid exchange catheter and process for unblocking a fluid exchange catheter**

(71) Applicant: Irras AB, 11145 Stockholm (SE)
(72) Inventor: The designation of the inventor has not yet been filed
(74) Representative: Bösl, Raphael Konrad

(57) **Abstract**

According to the invention, a fluid exchange catheter is provided, which catheter has a proximal end and a distal end, and comprises an obligatory lumen having a proximal end and a distal end, the distal end of the obligatory lumen having a closed shape, wherein a lumen wall of the obligatory lumen forms a distal wall portion at the distal end of the obligatory lumen, the distal wall portion comprising at least one lumen opening in the lumen wall. Furthermore, the catheter comprises at least one additional lumen having a proximal end and a distal end, the distal end being arranged inside the obligatory lumen remote from the distal end of the obligatory lumen such that fluid exiting the at least one additional lumen flows inside the distal end of the obligatory lumen. Here, the distal wall portion exhibits expansibility in at least an area surrounding the at least one lumen opening in order to provide an unblocking function for removing blockage of the catheter.

## Description

The proposed invention provides a fluid exchange catheter, for example for drug delivery into a body, i.e. human or animal tissue. Particularly, the proposed invention provides a fluid exchange catheter being able to prevent or remove blockage of the catheter, which can occur due to different causes, such as clogging by a blood clot, pus or the like. Further, the proposed invention provides a process for unblocking such a fluid exchange catheter. The primary field of application of the proposed invention involves tissue aberrations in deep tissue pathology or bone structure, but can be used in any other suitable surgical field.

For example, one particular field of application of the proposed fluid exchange catheter is the treatment of cerebral vasospasm. Since angiographic cerebral vasospasm is seen in 30-70% of patients with aneurysmal Subarachnoid Hemorrhage (SAH), more than one third of the patients with SAH develop clinically significant vasospasm as a leading morbidity and mortality factor for these patients. It is widely accepted that degradation products of blood are the causative factor of vasospasm. Since it could be determined that the amount of subarachnoid blood is correlated to the risk of vasospasm, reducing the subarachnoid clot burden at the time of surgery reduces the risk of vasospasm. It is also well known that clearing of blood from the cisterns during surgery helps reducing the incidence and severity of vasospasm. Here, however, it is highly difficult and invasive to clear all the blood sufficiently, since clearing the subarachnoid blood from cisterns following aneurysm rupture remains a technical challenge. Traditionally, neurosurgeons adopt a broad variety of irrigation methods and drains intra and post operative period. The respective risks are increasing intracranial pressure (ICP), inadequate clearance and infection. In particular, for the treatment of vasospasm, many theories have been proposed and various treatment regimens have been applied. So far, however, there is no existing method to clear the blood from subarachnoid spaces satisfactorily and with only low risk for the patient.

Traditionally, the tip of the catheter that is inserted in biological material, i.e. the patient's body, is called the distal tip or distal end of the catheter and the tip that stays outside of the biological material is called the proximal tip or proximal end of the catheter. Most of existing catheters provide a single lumen, through which lumen the user can alternatively infuse or aspirate liquids, such as solutions, blood, or the like. For example, in a clinical setting, the common intravenous catheter either aspirates blood samples, usually immediately after its insertion into the vein, or infuses solutions of drugs and/or nutrients usually for many hours or days following its insertion. With these catheters, it is possible to infuse or aspirate large quantities of liquids.

In the last years, it has been found that a concurrent fluid exchange can be desirable both for monitoring and therapeutic reasons. However, there are few catheters with multiple lumina, on the market at the present day, which can concurrently infuse and aspirate liquids sufficiently. For example, the microdialysis catheter after its introduction to a human or animal tissue is continuously perfused with liquid solutions from a pump connected to its proximal tip. The microdialysis catheter consists of a two lumen shaft where the distal tip is covered by means of a semipermeable membrane. Usually, the shaft is made of two concentric tubes, where the central tube is the efferent, and the peripheral tube is the afferent, part of the catheter. At the distal tip (i.e. inside the tissue), small solutes can cross the membrane by passive diffusion, hence fractions of the perfused liquid are infused to the tissue through the membrane, and extracellular fluid is aspirated through the same membrane and the efferent lumen. Microdialysis catheters and catheters similar to them, though, were particularly designed for tissue monitoring, and the above described concurrent infusion and aspiration takes place at a few microliter/minute rate flow range, using very small membrane pores for passive diffusion, and only for of small enough solutes.

For therapeutic applications, however, much greater liquid exchange rate and membranes or cages with big pores are required, so that it is possible to evacuate low viscosity liquids like blood clots, drug build-ups, pus and the like, that can block all existing catheters. Blockage constitutes a common problem of all kinds of existing catheters for biological fluids and occurs due to corking of biological material into their lumen's tip or its covering. For example, endotherapy catheter systems claim to possess the desired liquid exchange rate and blockage free operation through a moving part. As described in, for example, PCT/GR2004/000045 (PANOTOPOULOS CHRISTOS; 8 September 2003), endotherapy catheters consist of two concentrical lumen tubes, one infusing part and one aspirating part, connected properly to infusion and aspiration devices at their proximal tip, and having a filter, membrane, grid or mesh cage covering their distal tip, which catheters contain a hydrodynamically moving device for concurrent infusion and aspiration. The infusing tube is appropriately connected to a moving device that irrigates the surrounding of the catheter space, while simultaneously propels with its movement the aspiration through the outer tube.

As further state of the art, US 4 694 832 A, US 4 755 175 A, EP 0 251 512 A1 and EP 1 937 329 B1 are considered to be most relevant, as follows:
US 4 694 832 A describes a dialysis probe, primarily intended for insertion in biological tissues, for example brain tissue, the probe comprising a dialysis membrane and ducts for flow of the perfusion fluid over the membrane. Here, the dialysis membrane in such a probe can be surrounded by a mounting which supports and partially reveals the membrane, and which is more rigid than the membrane.
US 4 755 175 A shows a catheter intended for insertion into a canal or canal-like organ, for example into the urethra leading to the urinal bladder of a human being, for the purpose of emptying the contents of the urinal bladder of that person into the catheter. The catheter described in this document comprises a preferably flexible, tubular catheter body incorporating a urine inlet and provided with an insertion section together with means for holding the catheter within the urethra. The catheter body also includes a discharge section spaced from the insertion section and intended, for example, for connection to a urine collecting vessel. Here, the interior of the tubular insertion section is arranged to support a sieve or filter element, and means for creating turbulence in the incoming flow to the catheter body, so as to prevent blocking of the inlet opening.

Further, EP 0 251 512 A1 discloses an apparatus for removing obstructions from organs and bodily cavities. The apparatus, which can be connected to a catheter infusion port and a catheter aspiration port, can constantly vary in infusion and aspiration rates to maintain a preset pressure range. By this, the apparatus operates for dislodging and removing obstructions in bodily cavities or organs by both delivering and removing fluid thereto and is operable by continuous or intermittent perfusion of fluid over the preset pressure range. Therewith, the apparatus effects dissolution and removal of the obstructions.

Finally, in EP 1 937 329 B1, a fluid exchange catheter is described, comprising an inner lumen connected to an infusion mechanism, and an outer lumen connected to an aspiration mechanism, wherein both mechanisms can create programmable pressure changes in the lumens, and wherein the distal end of the inner lumen is disposed within the interior of the outer lumen such that a fluid exiting the inner lumen flows into the interior of the outer lumen.

Moreover, besides a concurrent fluid exchange for therapeutic reasons as described above, a concurrent fluid exchange in a catheter can also be desirable for monitoring reasons. In particular, it would be very helpful for any kind of medical procedure to be able to analyze aspirated fluid or tissue directly at the operation site and control the local infusion of drugs as a direct therapy based on the results of such an analysis without the risk of blood clogging or the like of the catheter. Such a possibility would not only be desirable at the moment of operation but also afterwards in case any harmful aftereffects of the medical procedure occur.

However, all of the above described further state of the art catheter variations are not able to prevent or remove a blockage of the catheter, at least not in a sufficient manner, especially in the case of a dimensionally small environment such as deep tissue pathology or bone structure. Therefore, the need for a new kind of catheter exists, in particular for a fluid exchange catheter which is able to generally provide a steady fluid exchange function without the risk of prolonged blockage of the catheter.

In order to achieve a solution for the above mentioned demand, the inventor of the present invention has developed a fluid exchange catheter which provides a stable fluid exchange performance without the risk of prolonged blockage of the catheter. In particular, the inventor developed a fluid exchange catheter having a proximal end and a distal end, the catheter comprising an obligatory lumen having a proximal end and a distal end, the distal end of the obligatory lumen having a closed shape, wherein a lumen wall of the obligatory lumen forms a distal wall portion at the distal end of the obligatory lumen, the distal wall portion comprising at least one lumen opening in the lumen wall, Furthermore, the catheter comprises at least one additional lumen having a proximal end and a distal end, the distal end being arranged inside the obligatory lumen remote from the distal end of the obligatory lumen, wherein the distal wall portion exhibits expansibility in at least an area surrounding the at least one lumen opening in order to provide an unblocking function for removing blockage of the catheter. Thereby, a stable fluid flow through the catheter can be achieved. Moreover, with the particular arrangement of the distal ends of the obligatory lumen and the at least one additional lumen, the distal end of the at least one additional lumen can be spaced apart from the distal end of the obligatory lumen towards the proximal end of the obligatory lumen by a certain distance, preferably by 20mm, more preferably by less than 10mm. Thereby, it is possible to achieve an interior lumen area inside the obligatory lumen between the distal end of the at least one additional lumen and the distal end of the obligatory lumen, which interior lumen space or interior lumen area can be used for, for example, drug dilution, fluid exchange, or the like.

Preferably, the expansibility of the distal wall portion is different than the expansibility of the at least one additional lumen, preferably the expansibility of the distal wall portion is greater than the expansibility of the at least one additional lumen. This can be achieved by providing the distal wall portion of the obligatory lumen in at least an area surrounding the at least one lumen opening with a certain expansibility, wherein particular elasticity characteristics of the distal wall portion at least in an area near the opening are required, which elasticity characteristics preferably originate from a particular choice of material of the distal wall portion of the outer lumen or areas thereof, such as polyurethane elastomers or silicone elastomers. Here, the distal wall portion is integrally formed with the obligatory lumen.

Further preferably, the at least one lumen opening comprises a particular shape, such as a slit shape, a cross shape, a star shape, a nozzle shape, an oval shape or the like. Also, the at least one opening can be formed in line with a combination of these shapes. In case of several openings, the different openings can have different shapes as described before, wherein, for example, several of the different openings can have a slit shape, such as a serpentine shape, and the other openings can have a star shape, depending on the demand. By means of such shapes, the distal wall portion can show a certain flexibility by structural design; for example, in the case of a cross shaped opening, the four flaps of the opening can be deflected when being obstructed by a clot or the like, thereby loosening the clot for removal. In doing so, the shape of the at least one lumen opening contributes to the expansibility of the distal wall portion.

Further, as alternative configurations of the fluid exchange catheter of the invention, the at least one additional lumen can be provided concentrically inside the obligatory lumen in order to provide a central infusion/aspiration location inside the distal wall portion, or the at least one additional lumen and the obligatory lumen are parallel tubes, adjacent or adjoining, at least at their distal ends, preferably produced by coextrusion or any other suitable production way for achieving such a lumen structure.

In general, with the fluid exchange catheter as described above, any mentioned lumen can be adapted for fluid infusion and/or fluid aspiration. For example, with a combination of obligatory lumen and at least one additional lumen, the obligatory lumen can be used either for fluid infusion and fluid aspiration or alternatively for both, and the at least one additional lumen can also be used either for fluid infusion and fluid aspiration or alternately for both, depending on the respective demands. Thereby, it is possible to deliver several different drugs to the same location in a body simultaneously or subsequently, which drugs would usually not be compatible with each other and could, thus, not be delivered in a sufficiently fast manner by one and the same lumen. Thus, a more complex treatment of, for example, vasospasm can be carried out immediately, while blood clots clogging the at least one opening can still be prevented or removed.

An unblocking of the catheter can also be effected by generally altering the width of the at least one lumen opening by changing the infusion and/or aspiration fluid pressure of the catheter, wherein the infusion and/or aspiration fluid pressure is different from an outer pressure acting on the outside of the distal wall portion of the obligatory lumen. Preferably, the width of the at least one lumen opening is enlarged when providing an infusion fluid pressure inside the distal end of the obligatory lumen higher than an outer pressure acting on the outside of the distal wall portion of the obligatory lumen, or the width of the at least one lumen opening is reduced , compared to a state of the at least one opening during infusion, when providing an aspiration fluid pressure inside the distal end of the obligatory lumen lower than an outer pressure acting on the outside of the distal wall portion of the obligatory lumen. Here, the width of the at least one opening reaches its maximum width during infusion. In general, an unblocking of the at least one opening can be achieved by enlarging or reducing the width of the at least one opening, for example by alternation of infusion and/or aspiration pressures or by mechanical operation. Such an alternation of the width of the at least one opening can preferably be achieved by providing at least the distal wall portion in at least an area surrounding the at least one lumen opening with a certain expansibility. For example, the catheter can be made of thermoplastic elastomers, such as polyurethane, and the tip can be made of silicone in order to provide the tip with more elasticity. Here, the distal wall portion is integrally formed with the obligatory lumen.

In the catheter, any obstruction of the at least one opening constitutes a constrained fluid flow through the at least one opening. Due to the expansibility of the area around the at least one opening, a so called balloon effect can be achieved in the area of the at least one opening due to increased infusion pressure or reduced aspiration pressure inside the obligatory lumen, which effect results in an expansion or shrinkage of the distal wall portion or at least in the area of the at least one opening. Thereby, any blockage of the at least one opening is loosened by the movement of its shape and, thus, can be flushed away or, if desired, aspirated by the obligatory lumen itself or the additional lumen. Also, with such a structure, it is possible to infuse required drugs or drug solutions, simultaneously or subsequently, to a particular location inside a body or to a target tissue, while any other substance at the same location, such as coagulated blood, toxic substances, or the like, can be evacuated, also simultaneously or subsequently, as desired.

In a further development of the catheter, the catheter can comprise at least two additional lumens, preferably wherein each lumen infuses at least one drug different from the at least one drug infused by another additional lumen. Here, the doctor could perform simultaneous administration of drugs that are not compatible with each other and actually need different infusion lumina, like, for example, a fluorouracil solution (cytotoxic chemotherapeutic drug) and a Morphine sulfate solution (analgesic). Similar effective drug combinations can be selected from a wide variety of analgesic, antibiotic, anti-inflamatory, cancer chemotherapy drugs, etc., as well as any drug eligible for direct administration to any pathology beneath the skin. In doing so, several different drugs can be delivered to the same location in a body simultaneously or subsequently, which drugs would usually not be compatible with each other and could, thus, not be delivered by one and the same lumen. Thus, a more complex treatment of, for example, vasospasm can be carried out immediately, while blood clots and the like can still be aspirated at the same time, for example by the obligatory lumen.

As a further feature for achieving the unblocking function of the catheter, a mechanical device can be provided, preferably inside the catheter, which mechanical device is connected to or integrally formed with at least a distal end of the obligatory lumen and/or the at least one additional lumen, the catheter thereby being adapted to transmit deflection force to the respective lumen(s) for generating a movement of at least the distal end of the same. With such a mechanism for distal end deflection movement, a fluid flow of any lumen can be altered, if desired. Also, the mechanical deflection movement itself can achieve a flexible deformation of the distal lumen end to be actuated, thereby loosening any blockage occurring at an opening of the respective lumen. Here, the mechanical device can be a guide wire or any device being able to transmit the necessary force to deflect the distal end of a lumen of a fluid exchange catheter.

In general, it is preferable with the fluid exchange catheter of the invention that any lumen is usable to remove substances, such as blood, pus, pathological tissue or toxic substances by aspiration. Preferably, the fluid exchange catheter comprises at least two lumens, wherein at least one of the lumens is adapted to remove these substances from the body. Thereby, simultaneous aspiration of hazardous material and infusion of required drugs can be achieved without the problem of clogging either the lumen used for infusion of the drugs or the lumen used for aspiration, by hazardous material, such as a blood clot. As further development, the fluid exchange catheter can be structured such that any one of the obligatory lumen and/or the at least one additional lumen is adapted to transmit fluid or guide minimally invasive medical equipment, such as a biopsy stylet or the like, or to guide several other, smaller lumens to the treatment site, also in alternation with fluid exchange. Thereby, the catheter of the invention can not only provide an alternating function of aspiration and infusion, but also an alternating function as fluid guiding means and equipment guiding means, i.e. a multifunctional catheter.

In order to be able to improve usage of the above described catheter, it can be advantageous to provide the catheter tip with an air sensor at the catheter's infusion part in order to use it to stop the infusion process for safety reasons, or in case of replacement of infusion liquid when consumed. Furthermore, it might be advantageous to further provide the catheter with an air sensor at the catheter's aspiration part in order to be able to stop aspiration for safety reasons in case no liquid aspires. Also, as a further advantageous feature, the catheter might be provided with a spectral sensor at the catheter's aspiration part, which sensor can be used to monitor the progress of a respective treatment including the catheter. Here, as an example for monitoring characteristics, dark red might indicate an old haemorrhage, light red might indicate an on-going haemorrhage, and no colour might indicate a complete evacuation of the haemorrhage.

The fluid exchange catheter according to the invention can also be used within a fluid exchange catheter system, for example in the form of an endoscopic system, wherein the proximal end of one lumen of the catheter is connected to at least one infusion device, such as a fluid container or a peristaltic pump or the like, for infusion of fluid into the body through the lumen, and the proximal end of another lumen is connected to at least one aspiration device, such as a fluid container or a peristaltic pump or valve or the like, for aspiration of fluid from the body through the obligatory lumen or the additional lumen(s), or vice versa. Thereby, different functions of the respective lumens can be achieved and controlled, such as, for example, infusion, aspiration, or alternating patterns of infusion and aspiration. Here, it is possible to provide several different kinds of infusion and/or aspiration devices or mechanisms, for example in order to achieve different infusion and/or aspiration flow speeds inside the catheter system, which can be necessary, for example, when infusing different drugs and/or carrying out simultaneous microdialysis processes with one and the same catheter system. Moreover, the infusion device and the aspiration device can be configured to create programmable changes of pressure in the lumens. Thereby, it is possible to carry out a process for unblocking a fluid exchange catheter, the fluid exchange catheter having a configuration as described above, and the process comprising the step of controlling infusion and/or aspiration through the obligatory lumen for creating a non-linear motion of the fluid flowing through the at least one opening. Here again, a non-linear motion denotes a fluid flow which is not linear, such as a swirling or vortexing flow, with which a blockage of the catheter, for example by a clot, can be loosened by the swirling flow impacting on the opening blocking clot and rocking the same. The catheter as used in the above described process can further comprise the step of controlling infusion and/or aspiration through the additional lumen for creating a non-linear motion of the fluid flowing through the at least one additional lumen, and into the distal end of the obligatory lumen. Here, any controlling step preferably includes adapting pressure rates of infusion and/or aspiration, and/or duration of infusion and/or aspiration times until unblocking of the catheter. As control in an unblocked catheter, pausing by switching off both infusion and aspiration during a cycle on infusion and aspiration will make the infusion input and aspiration output pressures at the proximal end of the catheter equalize to become equal to the ICP (or local tissue pressure). Also, in an unblocked catheter, switching off aspiration during one part of a cycle of infusion and aspiration will make the aspiration output pressure at the proximal end of the catheter stabilized to become equal to the ICP (or local tissue pressure), which in turn will increase slowly as the infusion progresses, and, in reverse, switching of infusion during aspiration will stabilize the pressure at the proximal end of the catheter to become equal to the ICP (or local tissue pressure), which in turn will decrease slowly as the aspiration progresses. As control, it is also possible to adjust the volume (flow x time) infused in a cycle of infusion and aspiration to keep the ICP (or local tissue pressure) constant. As an example of a treatment cycle, a pump used as infusion device infuses 1ml of liquid during 2 sec, with the aspiration closed (i.e. the pressure in the patient will increase) to step-up the tip pressure for unblocking, followed by 1 sec. of pause with the infusion turned off, wherein the aspiration is still closed. Then, the aspiration is turned on (infusion remains turned off) for 13 sec. to aspire hemorrhage & excess liquid, and to decrease the ICP (or local tissue pressure), followed by 4 sec. of pause with the aspiration turned off (infusion remains turned off) so that the intra-catheter pressure equilibrates with the ICP (or local tissue pressure). This provides for a cycle time of 20s for 1ml infused liquid or a flow of 180 ml/h when running the cycle repeatedly. Moreover, as an example of another treatment cycle using a drug having a half-life period of 4 minutes, the cycle could include an infusion time of 2 sec., a pause of 4 minutes, followed by an aspiration time of 1 minute and, finally, a pause of 58 seconds, so that the entire cycle involves a time span of 6 minutes. This provides for a cycle time of 6 minutes for lml infused liquid or a flow of 10 ml/h when running the cycle repeatedly. Further, as an example of a standby cycle, i.e. a cycle during which the catheter is kept unblocked without significantly treating the patient, a pump used as infusion device infuses 1ml of liquid during 2 sec. with the aspiration closed (i.e. the pressure in the patient will increase) to unblock the potentially clogged tip, followed by 4 sec. of pause with the infusion turned off (aspiration still closed). Then, the aspiration is turned on (infusion remains turned off) for 13 sec. to let out liquid and decrease the ICP (or local tissue pressure), followed by 221 sec. of pause with the aspiration turned off (infusion remains turned off) to equilibrate with the pressure outside of the catheter. This provides for a cycle time of 240s for 1ml infused liquid or a minimalist flow of 15 ml/h when running the cycle repeatedly.

In order to be able to carry out such a process, the catheter system as described above preferably comprises a mechanism adapted to activate the infusion device for a, infusion time period and at an infusion pressure higher than an outer pressure acting on the outside of the distal end of the obligatory lumen, wherein fluid exits the additional lumen through the opening in the distal end and enters the distal wall portion of the obligatory lumen. Here, the aspiration device is disabled during the infusion time period. In doing so, in particular by stopping or disabling aspiration, a sufficient distribution of infused liquid into the pathology can be achieved. Preferably, the controlling mechanism is adapted to activate the aspiration device for an aspiration time period and at an aspiration pressure lower than an outer pressure acting on the outside of the distal end of the obligatory lumen, wherein the controlling mechanism is adapted to disable the aspiration device during the infusion time period and the delay time period and to disable the infusion device during the aspiration time period and the delay time period. Thereby, a particular pattern of infusion and aspiration can be generated, achieving a smooth infusion process and a smooth aspiration process. Even more preferably, the infusion pressure is greater than the aspiration pressure, or the infusion time period is less than the aspiration time period, in order to support the respective executed infusion/aspiration-cycle.

Moreover, it is preferable for the catheter system that the controlling mechanism further comprises blockage means for blocking the at least one additional lumen and/or blockage means for blocking the obligatory lumen. For example, the blockage means for blocking the additional lumen can block the same for a first time interval followed by a second time interval of free flow of the fluid through the additional lumen. Also, the blockage means for blocking the obligatory lumen can block the same for a first time interval, optionally followed by a second time interval of free flow of the fluid through the obligatory lumen. Alternatively, the controlling mechanism can be adapted to infuse liquid through the additional lumen with a first pressure for a first time interval followed by a second time interval of stop, wherein the obligatory lumen is blocked during the first and second time intervals, and aspirate fluid through the obligatory lumen with a second pressure being lower than the first pressure during a third time interval, optionally followed by a fourth time interval during which all lumens are blocked, wherein the additional lumen is blocked during the third and fourth time interval. With such a structure and such a blocking of the respective lumens, any desired pattern of infusion and/or aspiration processes can be executed, depending on the demand. Here, it is also possible to connect the infusion device with the obligatory lumen and the aspiration device with the additional lumen.

The described catheter system can be adapted to be a self-regulating system by means of analyzing the aspirated fluid, e.g. in view of pH value, local pressure, concentration of ingredients, or the like, and carrying out the activation of the infusion device based on the results of the analysis. Thereby, a self-regulating system can be achieved, which system can provide support for a patient without the need of a doctor or within such small time periods in which a doctor would simply not be in the position to act on required changes of treatment or the like. Here, since the catheter system according to the invention has such a self-regulating function, it is possible for a patient to be treated without the need of a doctor or the like at all time. Thus, the inventive catheter system can be kept partly inside the body even after an operation procedure, for example in the time after operation when the patient is kept on the Intensive Care Unit (ICU). The use of such a system during the patient's stay on the ICU can significantly reduce the time of the patient on the ICU, according to recent studies carried out by the inventor by ∼30%.

In order to be able to achieve a multi-functional self-regulating endoscopic system, the same can comprise further components additionally to the fluid exchange catheter, such as one or several of the following components:
a) an optical fiber for observation and/or video recording;
b) an additional outer lumen for guiding a biopsy stylet, a microforceps, or the like, for biopsy or local tissue manipulation;
c) one or several electrodes for monopolar or bipolar coagulation;
d) a microdialysis catheter for biochemical and pharmacokinetic monitoring;
e) a sensor at the tip of the catheter, e.g. a pressure sensor, a temperature sensor, a pH value sensor, a sensor for a specific molecule or chemical compound, or the like;
f) a local irradiation probe.

With such a system, drugs can be infused while a microdialysis process can be carried out.

Further, instead of merely evacuating substances, it is also possible to gather tissue or fluid samples while administering drugs to the body or the target tissue.

As a particular example of such a system for the treatment of, for example, a solid malignant tumor, the endoscopic system of one example of the system is introduced into the inside of a pancreatic tumor under radiological inspection, wherein a catheter of this example consists of the following:
- a large lumen comprising a stylet or the like, for the introduction of the catheter probe into the pancreatic tumor under radiological inspection through a small incision of the abdomen's skin of the patient;
- an optical fiber, usable for direct inspection of the introduction of the catheter probe into the tumor and video-recording of the procedure in case radiological inspection is not desirable or enough for patients' safety;
- an infusion lumen for ringer's lactate solution inside an aspiration lumen also containing the above optical fiber, usable for washing the optical fiber's tip and clear the surgeon's view;
- a biopsy stylet or the like, usable for biopsies of the tumor after introduction i.e. after stylet's withdrawal;
- microsurgical instruments for local tissue manipulations apart from biopsy (like microforceps for tissue dissection, usable through the central lumen of the catheter probe;
- electrodes provided around the catheter probe, usable for bipolar coagulation of a bleeding during introduction of the catheter probe, during surgical manipulations or during withdrawal of it out of the patient's body, or usable for changing the physiological environment of the treatment site;
- a combination of three infusion lumina to be introduced into the large lumen after withdrawal of the biopsy stylet, usable for simultaneous administration of a cytotoxic chemotherapeutic drugs solution, an analgesic solution and an isotonic physiological solution for local drug concentration, temperature and pressure control. The above drug and physiological fluid infusions will be administrated for a period of several days and will be adjusted according to biochemical and physicochemical local parameters in order to enhance the drug's potential;
- a microdialysis catheter usable for local biochemical and pharmacokinetic monitoring;
- sensors at the tip of the catheter connected appropriately, usable for monitoring of local physicochemical parameters (like temperature, pH-value, etc). Such information, combined with the biochemical information provided by the microdialysis catheter, assist the doctor to optimize and individualize the chemotherapy treatment to be administered, according to the reaction of the malignant tissue to be treated. From time to time new biopsy samples of tissue can be extracted and analyzed, if needed. For this reason we can take the combination of three infusion lumina temporarily out in order to perform biopsy-sampling with appropriate stylets, microforceps or the like;
- a local irradiation probe, for example consisting at least in part of Iridium, usable through the central lumen for local irradiation of the tumor after chemotherapy and before withdrawal of the catheter probe out of the body;

After withdrawal of the catheter-probe, the patient will be examined radiologically in order to determine shrinkage of the tumor and organize operation or follow up treatment.

Another example is the introduction of the system's catheter into an intracranial or body septic collection, i.e. an abscess. An open operation can be substituted by a small cranioanatresis or skin puncture, respectively, just for the catheter's introduction. The pathological fluid collection, usually high pressurized and full of pus, will be gradually evacuated and the frequent antibiotic solution irrigation will achieve therapeutic drug concentration for days, which is especially needed for tissue with poor antibiotic drug access, like brain abscesses or osteomyelitic bones.

The present invention also relates to the following further aspects and embodiments:
According to one further aspect, the invention relates to a method of treating a patient comprising the step of administering to a patient in need thereof at least one drug by a catheter of the invention.

An even further aspect herein is a drug or drug combination for use in a method for treatment of the human or animal body by surgery or therapy, **characterized in that** a catheter of the invention is used in said method. Preferably, in the latter aspects, the drug or drug combination is administered to a patient by a catheter of the invention. Preferably, a drug combination comprises at least two drugs. A related aspect herein is a drug or drug combination for use in a diagnostic method practiced on the human or animal body, **characterized in that** a catheter of the invention is used in said method. In a particular embodiment, a diagnostic agent is administered to a patient by a catheter of the invention.

Generally herein, said drug(s) is/are not particularly limited. Drugs suitable for catheter administration are generally known to the skilled person, such as all eligible drugs for local infusion under the skin. In exemplary embodiments herein, the (at least one) drug is/are selected from the group consisting of antibiotics, anti-inflammatory drugs (e.g. corticosteroids or immune selective anti-inflammatory drugs), analgesics (e.g. non-steroidal anti-inflammatory drugs or opioids), chemotherapeutic drugs (e.g. alkylating agents, antimetabolites, anthracyclines, etc.), and hormones (e.g. insulin).

In particular embodiments, the catheter of the invention is used in the treatment of pain. Accordingly, in particular embodiments, the (at least one) drug is selected from analgesics. Analgesics as used herein may include narcotics.

In another embodiment, the catheter of the invention is used in the treatment of cancer. Non-limiting examples for cancer include a pancreatic tumor, a liver tumor and a brain tumor, such as craniopharyngioma. Accordingly, in particular embodiments, the (at least one) drug is selected from chemotherapeutic drugs, such as from cytostatic and cytotoxic chemotherapy drugs. Non-limiting example for such drugs include fluorouracil, methodrexate and a craniopharyngioma (intracavitary) chemotherapy drug.

In particular embodiments, the catheter of the invention is used for the removal of substances, such as undesired substances, from the body. Preferred examples of such substances are selected from the group consisting of blood, coagulated blood, blood clot(s) (thrombus/thrombi), pus, toxic substance(s), superfluous drug(s), and/or pathological tissue(s). Other examples of such substances include tissue, such as tissue sample(s).

In a preferred embodiment, the catheter of the invention is used in the treatment of cerebral vasospasm. In particular embodiments, the catheter of the invention is used in the treatment of aneurysmal subarachnoid hemorrhage (SAH). The latter embodiments may involve the clearing of subarachnoid blood and/or administration of at least one drug. A preferred non-limiting example for such drug is papaverine.

In one embodiment, the catheter of the invention is used as a self-regulating system, such as a self-regulating system not requiring the presence of a doctor and/or medical personnel, or a self-regulating system exceeding human capabilities as regards e.g. (rapid) treatment changes. In particular embodiments, the catheter of the invention is used in an ICU.

In particular embodiments, the catheter of the invention is used for monitoring a site within a patient's body. Said monitoring may e.g. include observation and/or video-recording.

In some embodiments herein, a method of treatment referred to herein comprises administration of at least one drug and/or a fluid by a catheter of the invention. In preferred embodiments said fluid is a physiological solution. That is, physiological solutions (which are generally not limited, and are well-known to the skilled person), such as ringer' lactate solution, may be administered (and optionally aspirated) by a catheter of the invention. In further preferred embodiments said fluid is a nutrient solution.

An even further aspect herein is a kit of parts comprising at least one catheter of the invention and at least one drug, preferably at least two drugs.

In general exemplary embodiments herein, "at least one drug" refers to one drug. In exemplary embodiments herein "at least one drug" refers to "at least two drugs". In exemplary embodiments herein, the terms "at least one drug" and "at least two drugs" refer to two, three, four, five, or six drugs, preferably two three or four drugs, preferably three drugs, preferably two drugs. In certain embodiments, the drugs used herein, such as the "at least two drugs", are incompatible. Incompatible drugs are not particularly limited - and are readily known to the skilled person, e.g. from standard handbooks on injectable drugs. In certain embodiments, incompatible drugs are not compatible for y-site injection and/or for injection in a single syringe. In exemplary embodiments herein, the "at least two drugs" are administered simultaneously. In exemplary embodiments herein, the "at least two drugs" are administered sequentially.

In certain embodiments herein, the at least one drug or at least two drugs may be a drug combination of any of the drugs described herein.

Generally herein, any combinations of the particular uses described are also envisaged.

The invention will now be described in more detail with reference to preferred embodiments of a fluid exchange catheter according to the invention and a process for using the same, and also to the accompanying drawings, in which
FIG. 1 illustrates a fluid exchange catheter system comprising a fluid exchange catheter according to a preferred embodiment of the invention;
FIGS. 2a to 2e show different alternatives of a lumen arrangement of the fluid exchange catheter of Fig. 1 in a cross section along line A-A in Fig. 1;
FIG. 3a shows an enlarged illustration of the preferred embodiment of the fluid exchange catheter of the invention, and FIG. 3b shows an alternative to the catheter as shown in Fig. 3 a; and
FIG. 4a shows another preferred embodiment of the fluid exchange catheter of the invention, and Figs. 4b and 4c show alternatives to the embodiment shown in Fig. 4a;

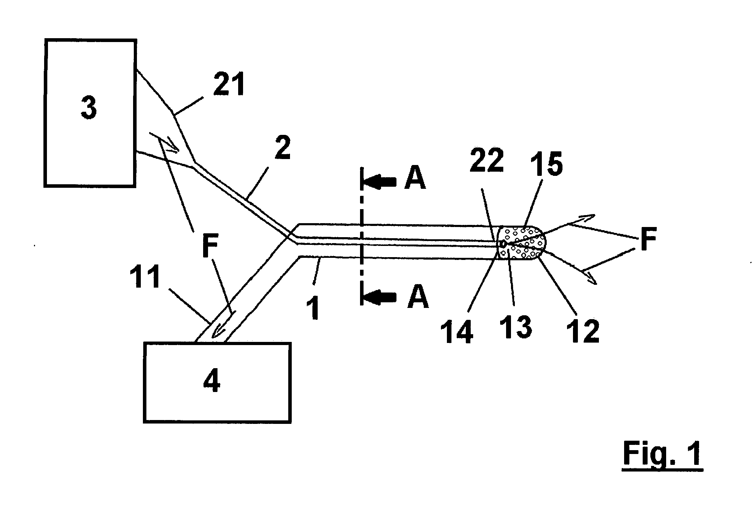

The catheter system as shown in Fig. 1 comprises a fluid exchange catheter according to a preferred embodiment of the invention, which consists of two lumen or lumen tubes 1 and 2, namely an obligatory lumen or outer lumen 1 and an additional lumen or inner lumen 2, wherein the additional lumen 2 is provided inside the obligatory lumen 1 in a concentrical manner. The additional lumen 2 is connected to an infusion device 3 such as a pump or a fluid container or the like, at its proximal tip or proximal end 21, and the obligatory lumen 1 is connected to an aspiration device 4 at its proximal tip or proximal end 11. Here, at the distal end or distal tip 22 of the additional lumen 2 and/or the distal end or distal tip 12 of the obligatory lumen 1, a covering in the form of a filter, nozzle, membrane, grid or mesh cage can be provided, or no covering over the distal ends 12, 22 can be provided at all. In this embodiment, the integrally formed distal end 12 of the obligatory lumen 1, more specifically a distal wall portion 13 of the obligatory lumen 1 marked by a separating line 14 is made of a different material (such as polyurethane elastomers or silicone elastomers) than the rest of the obligatory lumen 1, the material preferably exhibiting expansibility by having resilience characteristics in order to provide a ballooning effect when an opening 15 of the obligatory lumen 1 is clogged, for example by a blood clot or the like. Here, the distal end 12 of the obligatory lumen 1 is a generally closed end, i.e. shows a closed shape, for example in the form of a dome or the like, which comprises multiple lumen openings 15 in the lumen wall of the distal wall portion 13, thereby providing a fluid exchange surface of the catheter.

The infusion and aspiration devices 3, 4 periodically and/or continuously change liquid pressure gradients in the catheter system in order to create non-linear currents of fluid F that exits into the distal wall portion 13 of the obligatory lumen 1, i.e. inside the distal end 12 of the obligatory lumen 1, and wash the catheter's tips 12, 22 clean and keep the fluid exchange between the catheter and the tissue unobstructed, without the need of any moving parts. Nevertheless, the system as shown in Fig. 1 assures a flow rate that meets the needs for infusion and aspiration of the underlying pathology or the needs for monitoring and therapeutic or research protocol. The pressure differences in the system are created by any pattern of positive pressures of the infusion device 3, such as a pump or fluid container, and the accordingly synchronized pattern of negative pressures of the aspiration device 4, such as a pump or fluid container. Here the expression "pressures" always refer to the pressure at the catheter's tip surrounding tissue. The described system allows a fully and safely controllable infusion-aspiration rate and unobstructed fluid exchange. Alternatively, the obligatory lumen 1 can be connected to the aspiration device 4, and the additional lumen 2 can be connected to the infusion device 3.

Lots of patterns of pressure changes in the course of a process according to the invention can be applied depending on the underlying pathology or the research protocol. Here, such a process can consist of alternating steps of an infusion period of several seconds or even minutes with a particular infusion pressure, followed by an aspiration period of several seconds or even minutes with a particular aspiration pressure. In order to achieve such a pattern, it is desirable to provide blocking means, which are in the position to block a connection between the infusion device 3 and the additional lumen 2, or the connection between the aspiration device 4 and the obligatory lumen 1. For example, a blocking means can block the additional lumen 2 for a first time interval followed by a second time interval of free flow of the fluid through the additional lumen 2, or another blocking means can block the obligatory lumen 1 for a first time interval followed by a second time interval of free flow of the fluid through the obligatory lumen 1. Thereby, it is possible to achieve a pattern in which liquid is infused through the additional lumen 2 with a first pressure for a first time interval followed by a second time interval of stop, wherein the obligatory lumen 1 is blocked during the first and second time intervals, and in which fluid is aspirated through the obligatory lumen 1 with a second pressure being lower than the first pressure during a third time interval, wherein the additional lumen 2 is blocked during the third time interval, and optionally a fourth time interval during which all lumens 1 and 2 are blocked.

As one particular example, in one of the many possible system's versions regarding construction and operational mode, a peristaltic pump as infusion device 3 is programmed to infuse the liquid with a +200 mmHg pressure for 5 sec followed by 10 sec of stop, while the aspirating lumen tube 1 is blocked, and a peristaltic pump as aspiration device 4 is programmed to aspirate with a -100 mmHg pressure during the next 15 sec, while the infusing lumen tube 2 is blocked, in a 30 sec cycle of operation. Both these (infusion and aspiration) pressures at the ends of the system can be monitored to be kept synchronized within a predetermined range and phase difference and can be protected by alarms and automatic stops, such as the blocking means, whenever there is any system's dysfunction detection, by flow and/or pressure detector devices, which can be provided together with the blocking means, placed appropriately in the system for safety against over-infusion, over-aspiration etc..

Alternatively the infusion and aspiration devices 3, 4 of the system can be fluid containers, simply using the hydrostatic pressure forces created by their position relative to the catheter's tips 12, 22, as moving forces for the infused fluid to enter and the aspirated fluid to leave the tissue at the catheter's insertion site. For this version, it is possible to include one or several automatic button(s) as blocking means, programmed to compress the aspirating and/or infusing tube 1, 2 for 5 sec, followed by 5 sec of free flow of the aspirated (and infused) fluid or programmed for any other pattern of time intervals for free and blocked flow. Any mode of synchronized changes of pressure at any point is transferred directly to the infusing and aspirating tips of the catheter through the liquid column of infused or aspirated fluids. The catheter system as shown in Fig. 1 also comprises a bifurcation part of any configuration, in order to split the two opposite flows in two different lumens 1, 2.

Fluid, which can vary from distilled water to nutrient solutions including drugs, or also solid matter, for example drugs in any variety, supplied through the infusion device 3 to the additional lumen 2, reaches the distal end 12 of the obligatory lumen 1 where substance exchange occurs between the infused fluid and substances contained in the surrounding tissue's extracellular fluid. Then, the fluid mixture can be returned to an aspiration device or collection tank 4. Arrows F represent fluid streams or flows.

Alternatively to the above mentioned structure, it is possible to merely use one single obligatory lumen 1 comprising an opening, for example an oval slit opening, at its proximal end 11 and its distal end 12, respectively, wherein the proximal end 11 of the obligatory lumen 1 is alternately connected to the infusion device 3, the aspiration device 4 and/or a stop mechanism in between. Such an embodiment could be implemented by a device adapted for a pendulum movement, including two separate tubes corresponding to an aspiration pump or fluid container and an infusion pump or fluid container or connected thereto, respectively, wherein the pendulum device is constructed to connect the obligatory lumen 1 with one of the two tubes by a pendulum or oscillation movement. Here, due to the controlled movement of the pendulum and, thus, the controlled connection between the obligatory lumen 1 and one of the two tubes (or the stop in between), the single lumen catheter acts as infusion means or aspiration means depending on the respective connected opening of the pendulum, while any infusion or aspiration is stopped in between opening.

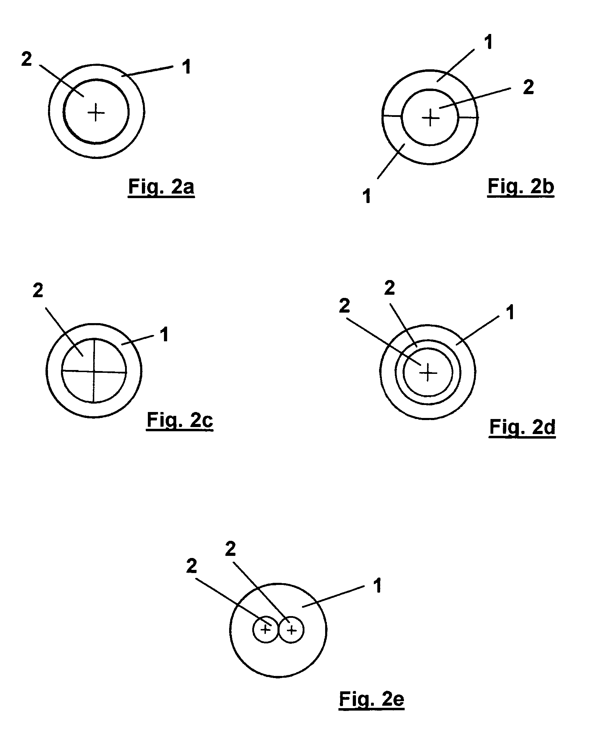

Fig. 2a shows a top view of a cross section along the line A-A in Fig. 1, in which it can be seen that the additional lumen 2 is concentrically arranged inside the obligatory lumen 1. Figs. 2b to 2e show alternative cross sections along the line A-A in Fig. 1 of alternative configurations of the catheter according to the preferred embodiment of the invention. In Fig. 2b, the obligatory lumen 1 is separated in half into two similar lumen spaces, leading to two obligatory lumens 1, 1, wherein the additional lumen 2 is arranged concentrically to both obligatory lumens 1, 1. In doing so, as an example, it becomes possible to infuse fluid through one of the obligatory lumens 1, 1, aspirate fluid through the other one, and use the additional lumen 2 for other purposes, such as guiding minimally invasive equipment. In Fig. 2c, the additional lumen 2 is separated into four equal additional lumen spaces, the entirety of which is arranged concentrically inside the single obligatory lumen 1. In doing so, as an example, several different incompatible drugs can be infused by the different additional lumen spaces, while the obligatory lumen 1 can be used for aspiration purposes. Fig. 2d shows an arrangement in which the inner additional lumen 2 is arranged concentrically inside the other, wherein both additional lumens 2, 2 are arranged concentrically inside the obligatory lumen 1. Finally, in Fig. 2e, there are two adjacent additional lumens 2, 2 arranged inside the obligatory lumen 1. These alternatives shown in Figs. 2a to 2e are only exemplary and can be altered or combined with each other.

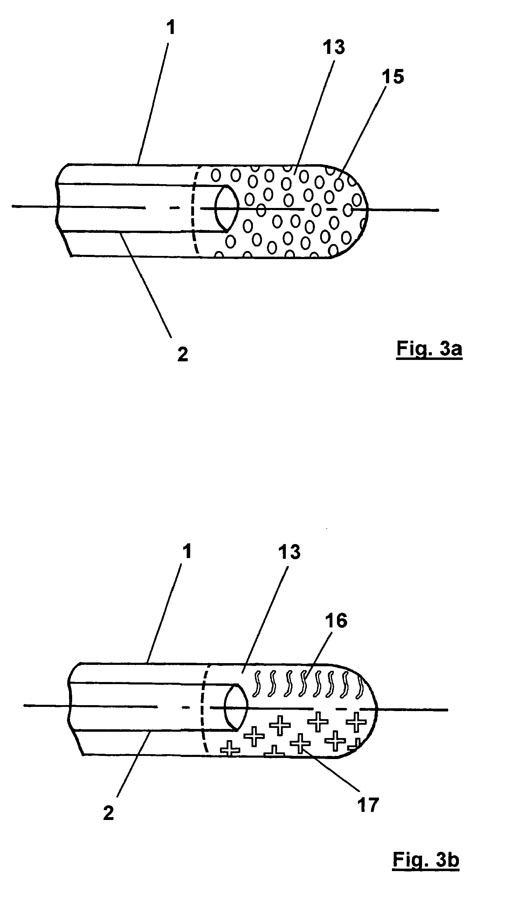

In Fig. 3a, the tip of the fluid exchange catheter according to the preferred embodiment of the invention is shown in detail. As mentioned before, the distal wall portion 13 of the obligatory lumen 1 is formed in a generally closed way, in the shape of a beacon or a dome or the like. Here, it can be seen that the openings 15 provided in the distal wall portion 13 of the obligatory lumen 1 have a generally circular shape. As generally described above, providing a pressure inside the distal wall portion 13 different from the outside of the distal wall portion 13 will result in a ballooning effect of the distal wall portion 13, wherein any blocking of one or several of the openings 15 will be loosened and, thus, can be removed. Fig. 3b shows an alternative distal end of the catheter, in which the openings are either shaped as serpentine slits 16 or in a cross shape 17, both shapes resulting in a structural expansibility of the distal wall portion 13.

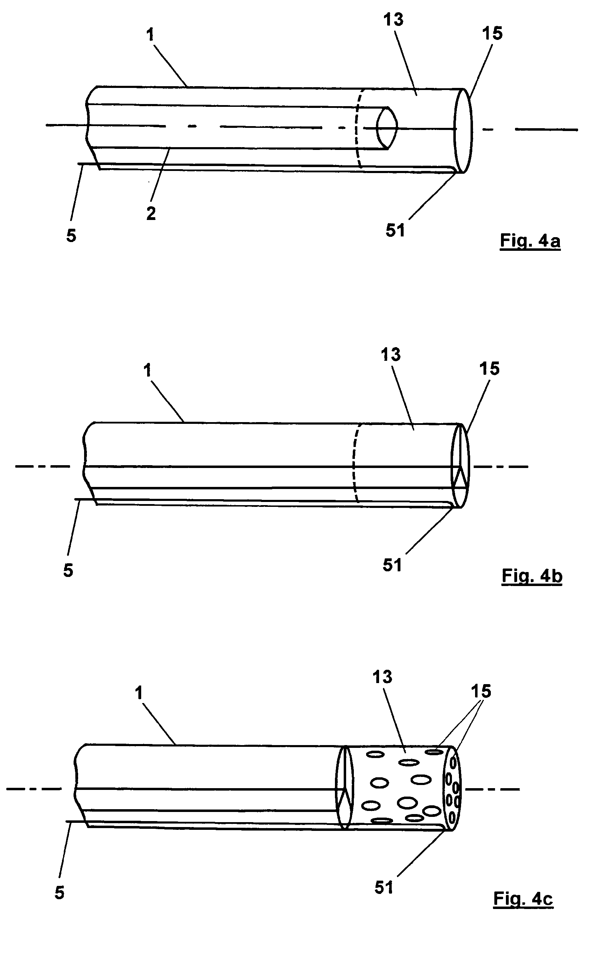

Fig. 4a shows another preferred embodiment of the fluid exchange catheter according to the invention. In this embodiment, a guide wire 5 is provided inside the obligatory lumen 1 and outside of the additional lumen 2, and is connected at a connection tip 51 with or within the distal wall portion 13 of the obligatory lumen 1. With such a mechanical device as the guide wire 5, it is possible by simply exerting a pulling force onto the guide wire 5 to pull the distal wall portion 13 and, thereby, to transmit a deflection force to the distal end 12 of the obligatory lumen 1 for generating a deflection of the part of the distal wall portion 13 connected with the connection tip 51 in order to deflect at least the distal end portion of the obligatory lumen 1. In doing so, a fluid flow streaming through the distal end of the obligatory lumen 1 can be altered, if desired. Also, the mechanical deflection movement itself can achieve a flexible deformation of the distal lumen end to be actuated, thereby loosening any blockage occurring at an opening 15 of the respective lumen to be deflected. As an alternative to the solution shown in Fig. 4a, Fig. 4b shows a fluid exchange catheter according to the invention, wherein the obligatory lumen 1 is divided into three separate lumen, i.e. a triple co-extruded lumen configuration, wherein the guide wire 5 is provided inside one of the three separate lumens. Here again, the guide-wire is connected at its connection tip 51 with the distal wall portion 13 of the obligatory lumen 1.

The structure of the obligatory lumen can also be as illustrated in Fig. 2c instead of the three-divided solution as illustrated in Fig. 4b. Further, Fig. 4c shows an alternative to the solution shown in Figs. 4a and 4b, wherein the obligatory lumen 1 is partly divided into three separate lumens, i.e. a triple co-extruded lumen configuration for the most part. Here, the division into three lumen ends before the distal end 12 of the obligatory lumen 1, such that a non-occupied or empty distal wall portion 13 is provided, wherein the guide wire 5 is provided inside one of the three separate lumens and is connected at its connection tip 51 with the hollow distal wall portion 13 of the obligatory lumen 1. In this alternative solution, the opening 15 as described above in view of 4a is generally closed, but the distal wall portion 13 comprises several smaller circular openings 15, similar to the openings as known from Fig. 3a as described above. The structure of the obligatory lumen 1 can also be as illustrated in Fig. 2c instead of the three-divided solution as illustrated in Fig. 4b. The technical effects of this preferred embodiment as shown in Figs. 4a, 4b and 4c are similar. Here, it is to be noted that, instead of the connection site between the distal wall portion 13 and the guide wire 5 as shown in the above described Figs. 4a to 4c, the connection tip 51 of the guide wire 5 can also be guided in the middle of the catheter and can be connected with a middle part of the obligatory lumen 1, for example at the connecting point of the three separate lumens as shown in Figs. 4b and 4c), or alternatively at any other part of the distal wall portion 13 suitable for deflection.

The construction material of the catheter as described above is in conformity to the norms and regulations existing for clinical and laboratory catheters, including biocompatibility issues etc..

## Claims

1. A fluid exchange catheter having a proximal end and a distal end, the catheter comprising:
an obligatory lumen (1) having a proximal end (11) and a distal end (12), the distal end (12) of the obligatory lumen (1) having a closed shape, wherein a lumen wall of the obligatory lumen (1) forms a distal wall portion (13) at the distal end of the obligatory lumen (1), the distal wall portion (13) comprising at least one lumen opening (15; 16, 17) in the lumen wall, and
at least one additional lumen (2) having a proximal end (21) and a distal end (22), the distal end (22) being arranged inside the obligatory lumen (1) remote from the distal end (12) of the obligatory lumen (1), wherein
the distal wall portion (13) exhibits expansibility in at least an area surrounding the at least one lumen opening (15; 16, 17) in order to provide an unblocking function for removing blockage of the catheter.

2. A fluid exchange catheter according to claim 1, wherein the expansibility of the distal wall portion (13) is different than the expansibility of the at least one additional lumen (2), preferably greater than the expansibility of the at least one additional lumen (2).

3. A fluid exchange catheter according to claim 1 or 2, wherein the at least one lumen opening (15; 16, 17) comprises a particular shape, such as a slit shape, a cross shape, a star shape, a nozzle shape, an oval shape or the like.

4. A fluid exchange catheter according to claim 3, wherein the shape of the at least one lumen opening (15; 16, 17) contributes to the expansibility of the distal wall portion (13).

5. A fluid exchange catheter according to any one of the preceding claims, wherein the at least one additional lumen (2) is provided concentrically inside the obligatory lumen (1).

6. A fluid exchange catheter according to any one of the preceding claims, wherein the at least one additional lumen (2) is provided parallel to the obligatory lumen (1) by coextrusion.

7. A fluid exchange catheter according to any one of the preceding claims, wherein any lumen (1, 2) can be adapted for fluid infusion and/or fluid aspiration.

8. A fluid exchange catheter according to claim 7, wherein an unblocking of the catheter is effected by altering the width of the at least one lumen opening (15; 16, 17) by changing the infusion and/or aspiration fluid pressure of the catheter, the infusion and/or aspiration fluid pressure being different from an outer pressure acting on the outside of the distal wall portion (13) of the obligatory lumen (1).

9. A fluid exchange catheter according to claim 8, wherein the width of the at least one lumen opening (15; 16, 17) is enlarged when providing an infusion fluid pressure inside the distal end (12) of the obligatory lumen (1) higher than an outer pressure acting on the outside of the distal wall portion (13) of the obligatory lumen (1), or
the width of the at least one lumen opening (15; 16, 17) is reduced when providing an aspiration fluid pressure inside the distal end (12) of the obligatory lumen (1) lower than an outer pressure acting on the outside of the distal wall portion (13) of the obligatory lumen (1).

10. A fluid exchange catheter according to any one of the preceding claims, wherein a mechanical device for unblocking of the catheter is provided inside the catheter, the mechanical device being connected to at least a distal end (12; 22) of any lumen (1, 2), the catheter thereby being adapted to transmit deflection force to the same for generating a movement of at least a distal end portion of said lumen (1, 2), preferably wherein the mechanical device is a guide wire or the like.

11. A fluid exchange catheter according to any one of the preceding claims, wherein at least one of the lumen (1, 2) infuses at least one drug, specifically wherein one lumen (1, 2) infuses at least one drug different from at least one drug infused by another lumen (2, 1).

12. A fluid exchange catheter according to any one of the preceding claims, wherein the obligatory lumen (1) and/or the at least one additional lumen (2) is usable to remove substances, such as blood, pus, pathological tissue or toxic substances.

13. A fluid exchange catheter according to any one of the preceding claims, wherein the obligatory lumen (1) and/or the at least one additional lumen (2) is adapted to transmit fluid or guide minimally invasive medical equipment.

14. Process for unblocking a fluid exchange catheter according to any one of the preceding claims, the process comprising the step of controlling infusion and/or aspiration through the obligatory lumen (1) for creating a non-linear motion of the fluid flowing through the at least one opening (15; 16, 17).

15. Process according to claim 14, wherein the process further comprises the step of controlling infusion and/or aspiration through the additional lumen (2) for creating a non-linear motion of the fluid flowing through the additional lumen (2).
